# EUROPÄISCHE PATENTSCHRIFT

(11) **EP 0 422 271 B1**
(45) Veröffentlichungstag und Bekanntmachung des Hinweises auf die Patenterteilung: **04.02.1998**
(21) Anmeldenummer: 89118911.0
(22) Anmeldetag: 11.10.1989
(51) Int. Cl.: A61N 1/365

(54) **In den Körper eines Lebewesens implantierbares medizinisches Gerät mit Mitteln zur Stimulation von Gewebekontraktionen**
Medical apparatus implantable in the body of a livingbeing with means for stimulating tissue contraction
Appareil médical implantable dans le corps d'un être vivant comportant des moyens de stimulation de contraction de tissus

(43) Veröffentlichungstag der Anmeldung: 17.04.1991
(73) Patentinhaber: Pacesetter AB, 171 95 Solna (SE)
(72) Erfinder: Engström, Jan, Dipl.Ing., S-163 55 Spanga (SE); Hedin, Asa, S-113 50 Stockholm (SE); Moberg, Lennart, Dipl.Ing., D-163 20 Spanga (SE)
(74) Vertreter: Lettström, Richard Wilhelm

(56) Entgegenhaltungen:
- EP-A- 318 304
- EP-A- 0 313 881
- EP-A- 0 326 629
- FR-A- 2 082 703
- US-A- 4 750 495
- El-Sherif, Samet:"CARDIAC PACING AND ELECTRO- physiology" 3rd edition, 1991, W.B. Saunders company, Philadelphia

## Beschreibung

Die Erfindung betrifft ein in den Körper eines Lebewesens implantierbares medizinisches Gerät mit Mitteln zum Detektieren spontaner Gewebekontraktionen, Mitteln zum Stimulieren von Gewebekontraktionen und Steuermitteln, die nach Ablauf eines jeweils durch die Detektion oder die Stimulation einer Gewebekontraktion in Gang gesetzten ersten Zeitintervalls, dessen Dauer einstellbar ist, die Mittel zum Stimulieren aktivieren, sofern nicht vor Ablauf des ersten Zeitintervalls eine ein neues erstes Zeitintervall in Gang setzende spontane Gewebekontraktion detektiert wird, die verhindern, daß vor Ablauf eines jeweils durch die Detektion oder Stimulation einer Gewebekontraktion in Gang gesetzten zweiten Zeitintervalls, dessen Dauer geringer als die des ersten Zeitintervalls ist, die Detektion einer spontanen Gewebekontraktion ein neues erstes Zeitintervall in Gang setzt, und die nach einer Detektion einer spontanen Gewebekontraktion während des zweiten Zeitintervalls ein neues zweites Zeitintervall in Gang setzen.

In der Druckschrift "DIALOG-Schrittmacher 728 - Gebrauchsanweisung", Oktober 1986, Druckzeichen: A91003-M3372-L777-01, Siemens-Elema AB, Solna, Schweden, ist ein derartiges Gerät beschrieben, das als Herzschrittmacher ausgebildet ist, bei dem die Dauer des ersten Zeitintervalls, das auch als Basis-Intervall bezeichnet wird, derjenigen Stimulationsfrequenz entspricht, mit der der Herzschrittmacher das Herz beim Ausbleiben von spontanen Herzschlägen stimuliert. Bei dem bekannten Herzschrittmacher sind sowohl die Dauer des ersten Zeitintervalls als auch die des zweiten Zeitintervalls durch einen Programmiervorgang unabhängig voneinander einstellbar. Dabei setzt sich das zweite Zeitintervall, das auch als Refraktärzeit bezeichnet wird, aus zwei Zeitabschnitten zusammen. Während des ersten Abschnittes des zweiten Zeitintervalls, der auch als absolute Refraktärzeit bezeichnet wird und dessen Dauer durch einen Programmiervorgang ebenfalls einstellbar ist, sind die Mittel zum Detektieren völlig unempfindlich gegenüber jeglichem Herzsignal. Hierdurch ist vermieden, daß nach Beaufschlagung des Herzens mit einem Stimulationsimpuls auftretende Gewebedepolarisationen zu Fehldetektionen führen, d.h. von den Mitteln zum Detektieren unzutreffenderweise als spontaner Herzschlag detektiert werden. In dem sich daran anschließenden zweiten Abschnitt des zweiten Zeitintervalls, der auch als relative Refraktärzeit bezeichnet wird, werden auftretende spontane Herzschläge zwar detektiert, allerdings beginnt kein neues Basis-Intervall zu laufen. Statt dessen wird die Refraktärzeit in ihrer vollen Länge erneut in Gang gesetzt. Schlägt also das Herz spontan so schnell, daß die spontanen Herzschläge jeweils innerhalb der relativen Refraktärzeit, also während des zweiten Abschnittes des zweiten Zeitintervalls auftreten, geht der bekannte Herzschrittmacher von dem VVI-Mode, in dem er normalerweise arbeitet, in den VOO-Mode über, der der asynchronen Betriebsart entspricht. Der bekannte Herzschrittmacher stimuliert das Herz dann unabhängig von den spontan auftretenden Herzschlägen mit einer Stimulationsfrequenz, die der Dauer des Basis-Intervalls entspricht. Dies ist grundsätzlich vorteilhaft, da hierdurch bestimmte Wiedereintrittstachykardien beendet werden können. Außerdem können während der relativen Refraktärzeit auftretende Fehldetektionen, wie sie z.B. bei einer zu hoch eingestellten Empfindlichkeit der Mittel zum Detektieren auftreten, kein neues Basis-Intervall in Gang setzen. Das Auftreten von Fehldetektionen kann also eine an sich erforderliche Stimulation des Herzens nicht verhindern. Unter diesem Gesichtspunkt ist es an sich zweckmäßig, bei dem bekannten Herzschrittmacher eine vergleichsweise lange Refraktärzeit einzustellen. Dies hat in der Praxis jedoch zur Folge, daß die spontane Herzschlagfrequenz unter körperlicher Belastung so weit ansteigen kann, daß der bekannte Herzschrittmacher, ohne daß hierfür ein physiologischer Grund vorliegt, in seine asynchrone Betriebsart, also in den VOO-Mode übergeht. Das Herz des Lebewesens wird dann also stimuliert, ohne daß dies tatsächlich erforderlich ist. Dies kann zu Herzrhythmustörungen führen. Jedenfalls empfindet das den Herzschrittmacher tragende Lebewesen körperliches Unbehagen. Nur ein sehr erfahrener Arzt ist in der Lage, unter Berücksichtigung der individuellen Bedürfnisse und Eigenheiten des jeweiligen Patienten die Dauer der Refraktärzeit für ein bestimmtes Basis-Intervall so zu programmieren, daß einerseits schädliche Auswirkungen von Fehldetektionen und von außen eingestrahlten Störungen vermieden sind und andererseits ein physiologisch unbegründeter Übergang des Herzschrittmachers in seine asynchrone Betriebsart ausgeschlossen ist. Die vorstehenden Ausführungen gelten sinngemäß auch für im SSI- oder DDD-Mode arbeitende Herzschrittmacher, bei denen die Gefahr des physiologisch unbegründeten Übergangs in den SOO- bzw. AOO-Mode besteht.

In der Druckschrift EP-A2-0 318 304 ist noch ein derartiges Gerät beschrieben, und zwar ein Herzschrittmacher, bei dem ein zweites Zeitintervall variiert ist folgend der gemessenen Zeit zwischen einem ventrikularen Ereignis und dem nächsten atriellen detektierten Ereignis. Ein Teil dieses zweiten Zeitintervalls ist in einem ersten Detektierungsteil T1 und einem zweiten Detektierungsteil T2 eingeteilt, wobei atrielle Detektierungsereignisse, die innerhalb des ersten Detektierungsteils T1 fallen ignoriert werden können, während atriellen Detektierungsereignisse die innerhalb des zweiten Detektierungsteils T2 fallen, unter anderem zur Ermittlung eines neuen Zeitintervalls führen. Der erste Detektierungsteil T1 kann nach dieser Druckschrift als eine postventrikuläre atrielle Refraktärperiode betrachtet werden, während welcher der Herzschrittmacher aber auch in Detektierungslage eingestellt ist.

Ein anderer Herzschrittmacher mit veränderlicher Hystereseintervall abhängig von einem variablen Basis-Intervall ist in der Druckschrift EP 0 318 304 beschrieben.

Der Erfindung liegt die Aufgabe zugrunde, ein Gerät und ein Verfahren der eingangs genannten Art so auszubilden, daß sichergestellt ist, daß die Dauer des zweiten Zeitintervalls einerseits zur Vermeidung von schädlichen Auswirkungen von Fehldetektionen ausreichend lang und andererseits zur Vermeidung an sich unnötiger Stimulationen von Gewebekontraktionen, insbesondere bei hoher körperlicher Aktivität des Lebewesens, ausreichend kurz ist.

Nach einem ersten Lösungsprinzip wird der ein Gerät betreffende Teil der Aufgabe erfindungsgemäß dadurch gelöst, daß die Steuermittel die Dauer des zweiten Zeitintervalls in Abhängigkeit von der eingestellten Dauer des ersten Zeitintervalls derart einstellen, daß die Dauer des zweiten Zeitintervalls um ein definiertes Maß geringer als die eingestellte Dauer des ersten Zeitintervalls ist. Im Gegensatz zum Stand der Technik, wo sowohl die Dauer des ersten als auch die Dauer des zweiten Zeitintervalls vom Arzt festgelegt und eingestellt werden muß, genügt es im Falle der Erfindung also, wenn der Arzt die Dauer des ersten Zeitintervalls festlegt. Die Dauer des zweiten Zeitintervalls wird dann durch die Steuermittel in Abhängigkeit von der eingestellten Dauer des ersten Zeitintervalls so eingestellt, daß die Dauer des zweiten Zeitintervalls um ein definiertes Maß geringer als die des ersten Zeitintervalls ist. Bei geeigneter Wahl des definierten Maßes ist also sichergestellt, daß einerseits das zweite Zeitintervall ausreichend lang ist, um zu verhindern, daß beispielsweise Fehldetektionen ein neues erstes Zeitintervall in Gang setzen. Andererseits ist es bei geeigneter Wahl des definierten Maßes praktisch ausgeschlossen, daß das Gerät bei hoher körperlicher Aktivität des Lebewesens in seiner asynchrone Betriebsart übergeht, ohne daß hierfür ein physiologischer Anlaß besteht. Außerdem ist gewährleistet, daß bei einer Umprogrammierung der Dauer des ersten Zeitintervalls nach erfolgter Implantation des Gerätes stets die erforderliche Korrektur der Dauer des zweiten Zeitintervalls erfolgt, ohne daß es hierzu eines Eingriffs des behandelnden Arztes bedarf.

Nach der Erfindung wird der ein Gerät betreffende Teil der Aufgabe gemäß einem zweiten Lösungsprinzip dadurch gelöst, daß Mittel zur Ermittlung einer an die jeweilige körperliche Aktivität des Lebewesens angepaßten Kontraktionsfrequenz vorgesehen sind und daß die Steuermittel die Dauer des zweiten Zeitintervalls derart einstellen, daß diese einerseits in ihrem zeitlichen Verlauf dem zeitlichen Verlauf der der ermittelten angepaßten Kontraktionsfrequenz entsprechenden Periodendauer wenigstens im wesentlichen folgt und geringer als diese Periodendauer ist und andererseits stets geringer als die eingestellte Dauer des ersten Zeitintervalls ist. Im Gegensatz zum Stand der Technik, wo die Dauer des zweiten Zeitintervalls zwar normalerweise programmierbar ist, während des normalen Betriebes des Gerätes jedoch einen festen Wert besitzt, ändert sich die Dauer des zweiten Zeitintervalls im Falle der Erfindung in Abhängigkeit von der ermittelten an die körperliche Aktivität des Lebewesens angepaßten Kontraktionsfrequenz und zwar derart, daß sie in ihrem zeitlichen Verlauf der Periodendauer folgt, die der jeweils ermittelten angepaßten Kontraktionsfrequenz entspricht, und geringer als diese ist. Hierdurch ist sichergestellt, daß einerseits das zweite Zeitintervall ausreichend lang ist, um zu verhindern, daß Fehldetektionen ein neues erstes Zeitintervall in Gang setzen. Andererseits ist es infolge des Umstandes, daß sich die Dauer des zweiten Zeitintervalls an der der jeweiligen körperlichen Aktivität des Lebewesens angepaßten Kontraktionsfrequenz orientiert, ausgeschlossen, daß das Gerät bei hoher körperlichen Aktivität des Lebewesens in seine asynchrone Betriebsart übergeht, ohne daß hierfür ein physiologischer Anlaß besteht. Diese Gefahr wird nochmals dadurch verringert, daß die Dauer des zweiten Zeitintervalls geringer als die der der ermittelten angepaßten Kontraktionsfrequenz entsprechende Periodendauer ist.

Eine bevorzugte Variante der Erfindung sieht vor, daß die Mittel zur Ermittlung der angepaßten Kontraktionsfrequenz eine Sensoreinrichtung zur Bildung eines der körperlichen Aktivität entsprechenden Signales aufweisen und daß die Steuermittel derart ausgebildet sind, daß sie anhand des Signales der Sensoreinrichtung die angepaßte Kontraktionsfrequenz ermitteln. Geeignete Sensoreinrichtungen und Methoden zur Ermittlung einer an die körperliche Aktivität des Lebewesens angepaßten Kontraktionsfrequenz sind beispielsweise in der EP-A-0 080 348 am Beispiel eine piezoelektrischen Sensors und in der US-PS 4 543 954 am Beispiel eines Temperatursensors beschrieben.

Eine bevorzugte Ausführungsform der Erfindung sieht vor, daß die Mittel zur Ermittlung der angepaßten Kontraktionsfrequenz Mittel zum Bestimmen der Folgefrequenz unmittelbar aufeinanderfolgender spontaner Gewebekontraktionen aufweisen und daß die Steuermittel die bestimmte Folgefrequenz als ermittelte angepaßte Kontraktionsfrequenz berücksichtigen. Diese Ausführungsform ist insbesondere für solche Therapiefälle vorgesehen, bei denen während längerer Perioden physiologisch korrekte spontane Gewebekontraktionen des Lebewesens auftreten, die dann unmittelbar ein Maß für die der jeweiligen körperlichen Aktivität angepaßte Kontraktionsfrequenz darstellen.

Gemäß einer vorteilhaften Variante der Erfindung ist vorgesehen, daß die Steuermittel derart ausgebildet sind, daß sie die Dauer des zweiten Zeitintervalls so einstellen, daß diese um ein definiertes Maß geringer als die der der ermittelten angepaßten Kontraktionsfrequenz entsprechende Periodendauer ist. Eine weitere Variante der Erfindung sieht vor, daß das definierte Maß einstellbar ist. Der behandelnde Arzt ist also in der Lage, das definierte Maß den Bedürfnissen des jeweiligen Lebewesens und des Behandlungsfalles entsprechend einzustellen, was zweckmäßigerweise dadurch geschieht, daß das Gerat in an sich bekannter Weise auf nicht-invasivem Wege programmierbar ist, so daß der Arzt das definierte Maß auch nach der Implantation des Gerätes verändern kann.

Für beide Lösungsprinzipien kann vorgesehen sein, daß das definierte Maß einem definierten Prozentsatz, beispielsweise 20%, der Dauer des ersten Zeitintervalls bzw. der der ermittelten angepaßten Kontraktionsfrequenz entsprechenden Periodendauer entspricht. Es kann aber auch vorgesehen sein, daß das definierte Maß einem definierten Betrag der Frequenz entspricht, um den die der Dauer des zweiten Zeitintervalls entsprechende Frequenz größer als die der Dauer des ersten Zeitintervalls entsprechende Frequenz bzw. die ermittelte angepaßte Kontraktionsfrequenz ist. Es wird dann also stets eine Dauer des zweiten Zeitintervalls eingestellt, der eine Frequenz entspricht, die beispielsweise um 10 Hz größer als die jeweils ermittelte angepaßte Kontraktionsfrequenz bzw. die der Dauer des ersten Zeitintervalls entsprechende Frequenz ist. Hierdurch wird der Vorteil erzielt, daß die Größe des definierten Maßes für den behandelnden Arzt besonders anschaulich ist. Um eine nochmals erhöhte Anschaulichkeit zu gewährleisten, empfiehlt es sich, die Steuermittel weiter derart auszubilden, daß der definierte Betrag der Frequenz in einer in dem jeweiligen medizinischen Gebiet gängigen, eventuell von Hz abweichenden Dimension angegeben werden kann. Im Falle der Kardiologie sollte beispielsweise die Einheit Herzschläge pro Minute (bpm) gewählt werden.

Eine weitere bevorzugte Ausführungsform der Erfindung sieht vor, daß die Steuermittel derart ausgebildet sind, daß sie die Dauer des ersten Zeitintervalls so einstellen, daß diese der der ermittelten angepaßten Kontraktionsfrequenz entsprechenden Periodendauer entspricht. Da im Rahmen der Erfindung die an die jeweilige körperliche Aktivität des Lebewesens angepaßte Kontraktionsfrequenz ohnehin ermittelt wird, ist es also mit minimalem Aufwand möglich, die Dauer des ersten Zeitintervalle so einzustellen, daß sichergestellt ist, daß die Stimulation von Gewebekontraktionen im Bedarfsfall mit einer an die jeweilige körperliche Aktivität des Lebewesens angepaßten Stimulationsfrequenz erfolgt. Gemäß einer Ausführungsform der Erfindung ist das Gerät als Herzschrittmacher ausgebildet, wobei die Mittel zum Detektieren spontane Herzmuskelkontraktionen detektieren, die Mittel zum Stimulieren Herzmuskelkontraktionen stimulieren und die Mittel zur Ermittlung einer der jeweiligen körperlichen Aktivität des Lebewesens angepaßten Kontraktionsfrequenz die der jeweiligen körperlichen Aktivität des Lebewesens angepaßte Herzschlagfrequenz bzw. die spontane Herzschlagfrequenz des Lebewesens ermitteln.

Zwei erfindungsgemäße Lösungen des ein Verfahren betreffenden Teiles der Aufgabe sind in den kennzeichnenden Teilen der Patentansprüche 11 und 12 angegeben. Die Wirkungsweise und die Vorteile der erfindungsgemäßen Verfahren ergeben sich aus der vorstehenden Erläuterung des erfindungsgemäßen Gerätes.

Ein Ausführungsbeispiel der Erfindung wird nachfolgend anhand der beigefügten Zeichnungen am Beispiel eines Herzschrittmachers erläutert. Es zeigen:
- Fig. 1: ein Blockschaltbild eines erfindungsgemäßen Herzschrittmachers, und
- Fig. 2 und 3: zwei unterschiedliche Betriebsweisen des erfindungsgemäßen Herzschrittmachers verdeutlichende Diagramme.

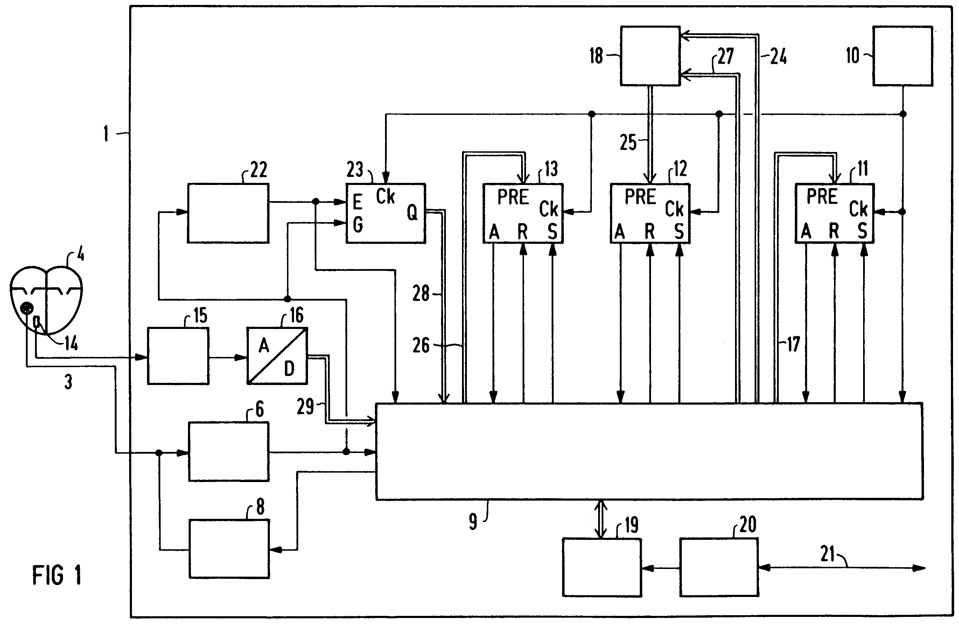

In der Fig. 1 ist ein erfindungsgemäßer Herzschrittmacher dargestellt, der in der VVI-Betriebsart arbeitet. Der Herzschrittmacher, dessen Elektronik in einem hermetisch dichten, implantierbaren Gehäuse 1 angeordnet ist, steht über eine endokardielle Elektrode 3 mit dem schematisch angedeuteten Herz 4 des den Herzschrittmacher tragenden Patienten in Verbindung. Dabei ist die Elektrode 3 durch das Venensystem zu dem Herzen 4 des Patienten geführt und in dem rechten Ventrikel des Herzens 4 verankert.

Die Elektrode 3 steht mit einer Detektoreinrichtung 6 in Verbindung, die zur Detektion spontaner Herzmuskelkontraktionen im Bereich des Ventrikels dient. Die Elektrode 3 ist außerdem mit einem Stimulationsimpuls-Generator 8 verbunden, der dazu dient, Herzmuskelkontraktionen im Bereich des Ventrikels zu stimulieren.

Das Zusammenwirken der Detektoreinrichtung 6 und des Stimulationsimpuls-Generators 8 mit dem Herz 4 wird durch eine Steuerlogik 9 gesteuert, an die ein Taktgenerator 10, ein PP-Zähler 11, ein R-Zähler 12 und ein RA-Zähler 13 angeschlossen sind. Bei den Zählern 11, 12, 13 handelt es sich um sogenannte PRESET-Zähler. Ein derartiger Zähler, dem Taktimpulse über einen entsprechenden Eingang Ck zugeführt sind, zählt während eines Zählvorganges jeweils eine Anzahl von Taktimpulsen ab, die durch einem mit PRE bezeichneten Eingang des Zählers zugeführte Daten bestimmt ist. Bei Erreichen der bestimmten Anzahl von Taktimpulsen gibt der Zähler an seinem Ausgang A ein entsprechendes Signal ab. Ein derartiger Zähler beginnt einen Zählvorgang, wenn einem mit S bezeichneten Eingang ein Startimpuls zugeführt wird. Der Zählvorgang wird abgebrochen und/oder der Zähler zurückgestellt, wenn einem Eingang R ein Rückstellimpuls zugeführt wird. Im Falle des beschriebenen Herzschrittmachers erhalten die Zähler 11, 12, 13 ihre Start- und Rückstellimpulse von der Steuerlogik 9. Ihre Taktimpulse erhalten die Zähler 11, 12, 13 von dem Taktgenerator 10.

Im folgenden wird die Arbeitsweise des Herzschrittmachers nur in dem Umfang beschrieben, wie dies im Zusammenhang mit der vorliegenden Erfindung erforderlich ist. Bezüglich weiterer Informationen wird auf die einschlägige Literatur, z.B. die Druckschriften "Pulse Generator 704 - Physican's Manual" Siemens-Elema AB, Solna, Schweden, März 1985 und "DIALOG Schrittmacher 728 - Gebrauchsanweisung", Siemens-Elema AB, Solna, Schweden, Oktober 1986, verwiesen.

Wird mittels der Detektoreinrichtung 6 eine spontane Herzmuskelkontraktion im Bereich des Ventrikels detektiert, gelangt von der Detektoreinrichtung 6 ein entsprechendes Signal zu der Steuerlogik 9. Diese startet daraufhin den PP-Zähler 11, der wie erwähnt seine Taktimpulse von dem Taktgenerator 10 erhält. Bei dem Taktgenerator 10 handelt es sich um einen Oszillator, beispielsweise einen Quarz-Oszillator, der mit einer definierten Taktfrequenz Taktimpulse abgibt, die auch der Steuerlogik 9 zugeführt sind. Hat der PP-Zähler 11 eine durch seinem Eingang PRE von der Steuerlogik 9 über eine Datenleitung 17 zugeführten Daten in noch zu beschreibender Weise programmierbare oder einstellbare Anzahl von Taktimpulsen abgezählt, die der Dauer eines ersten Zeitintervall, dem sogenannten Basis- oder auch PP-Intervall, entspricht, gelangt von seinem Ausgang ein entsprechendes Signal an die Steuerlogik 9. Diese aktiviert daraufhin den Stimulationsimpuls-Generator 8 zur Abgabe eines Stimulationsimpulses, der die Stimulation einer Herzmuskelkontraktion im Bereich des Ventrikels bewirkt. Gleichzeitig stellt die Steuerlogik 9 den PP-Zähler 11 zurück und startet diesen erneut. Die Abgabe eines Stimulationsimpulses mittels des Stimulationsimpuls-Generators 8 unterbleibt jedoch, wenn während des Basis-Intervalls mittels der Detektoreinrichtung 6 eine spontane Herzmuskelkontraktion im Bereich des Ventrikels detektiert wird. Geht der Steuerlogik 9 von der Detektoreinrichtung 6 ein entsprechendes Signal zu, stellt sie den PP-Zähler 11 zurück und startet ihn erneut.

Es wird also deutlich, daß das Herz, sobald die Folgefrequenz der spontanen Herzschläge unter eine Folgefrequenz abfällt, die der Dauer des Basis-Intervalls entspricht, in einer solchen Weise stimuliert wird, daß wenigstens eine Herzschlagfrequenz vorliegt, die der Dauer des Basis-Intervalls entspricht.

Allerdings wird nicht durch jede Detektion während des Basis-Intervalls in der beschriebenen Weise der PP-Zähler 11 zurückgestellt und erneut gestartet. Vielmehr ist es hierzu erforderlich, daß ein zweites Zeitintervall, die sogenannte Refraktärzeit, abgelaufen ist. Die Refraktärzeit wird mittels des R-Zählers 12 ermittelt. Wird mittels der Detektoreinrichtung 6 eine spontane Herzmuskelkontraktion im Bereich des Ventrikels detektiert und gelangt ein entsprechendes Signal von der Detektoreinrichtung 6 zu der Steuerlogik 9 oder aktiviert die Steuerlogik 9 den Stimulationsimpuls-Generator 8 zur Abgabe eines Stimulationsimpulses, startet die Steuerlogik 9 den R-Zähler 12, der seine Taktimpulse ebenso wie der PP-Zähler 11 von dem Taktgenerator 10 erhält. Hat der R-Zähler 12 eine durch seinem Eingang PRE zugeführte Daten in noch zu beschreibender Weise einstellbarer Anzahl von Taktimpulsen abgezählt, die der Dauer der Refraktärzeit entspricht, gelangt von seinem Ausgang ein entsprechendes Signal an die Steuerlogik 9. Erst nach Eintreffen dieses Signals führt eine während des laufenden Basisintervalls mittels der Detektoreinrichtung 6 detektierte spontane Herzmuskelkontraktion dazu, daß die Steuerlogik 9 den PP-Zähler 11 zurückstellt und erneut startet. Wird dagegen mittels der Detektoreinrichtung 6 eine spontane Herzmuskelkontraktion detektiert, bevor das das Ende der Refraktärzeit anzeigende Signal von dem R-Zähler 12 zu der Steuerlogik 9 gelangt ist, setzt diese den R-Zähler 12 zurück und startet ihn erneut, so daß die Refraktärzeit von neuem zu laufen beginnt. Da in diesem Falle ein Zurücksetzen und erneutes Starten des PP-Zählers 11 durch die Steuerlogik 9 unterbleibt, aktiviert die Steuerlogik den Stimulationsimpuls-Generator 8 nach Ablauf des Basis-Intervalls zur Abgabe eines Stimulationsimpulses, es sei denn, daß nach Ablauf der erneut in Gang gesetzten Refraktärzeit und vor Ablauf des laufenden Basis-Intervalls erneut eine spontane Herzmuskelkontraktion mittels der Detektoreinrichtung 6 detektiert wird.

Das Zurücksetzen und erneute Starten des R-Zählers 12 erfolgt übrigens nur während eines zweiten Abschnittes der Refraktärzeit, der auch als relative Refraktärzeit bezeichnet wird. Der der relativen Refraktärzeit vorangehende erste Abschnitt der Refraktärzeit wird als absolute Refraktärzeit bezeichnet.

Während der absoluten Refraktärzeit sind keinerlei Detektionen möglich. Dies wird im Falle des beschriebenen Ausführungsbeispieles dadurch erreicht, daß die Steuerlogik 9 während der absoluten Refraktärzeit von der Detektoreinrichtung 6 stammende, auf eine Detektion hindeutende Signale ignoriert. Die absolute Refraktärzeit wird mittels des RA-Zählers 13 ermittelt. Wird mittels der Detektoreinrichtung 6 eine spontane Herzmuskelkontraktion im Bereich des Ventrikels detektiert und gelangt ein entsprechendes Signal von der Detektoreinrichtung 6 zu der Steuerlogik 9 oder aktiviert die Steuerlogik 9 den Stimulationsimpuls-Generator 8 zur Abgabe eines Stimulationsimpulses, startet die Steuerlogik 9 den RA-Zähler 13, der seine Taktimpulse ebenso wie der PP-Zähler 11 und der R-Zähler 12 von dem Taktgenerator 10 erhält. Hat der RA-Zähler 13 eine durch seinem Eingang PRE über die Datenleitung 26 von der Steuerlogik 9 zugeführte Daten in noch zu beschreibender Weise programmierbare Anzahl von Taktimpulsen abgezählt, die der Dauer der absoluten Refraktärzeit entspricht, gelangt von seinem Ausgang ein entsprechendes Signal an die Steuerlogik 9. Dies bedeutet, daß die absolute Refraktärzeit abgelaufen ist und die relative Refraktärzeit läuft, was zur Folge hat, daß die Steuerlogik 9 von der Detektoreinrichtung 6 stammende Signale akzeptiert. Während der absoluten Refraktärzeit von der Detektoreinrichtung 6 zu der Steuerlogik 9 gelangende Signale führen also weder zu einem Zurücksetzen und erneuten Starten des PP-Zählers 11 noch des R-Zählers 12.

Es wird also deutlich, daß der Herzschrittmacher in die asynchrone Betriebsart (VOO-Mode) übergeht, in der er das Herz unabhängig von eventuellen Detektionen spontaner Herzschläge mit einer Stimulationsfrequenz stimuliert, die der eingestellten bzw. programmierten Dauer des Basis-Intervalls entspricht, wenn die Detektion aufeinander folgender spontaner Herzschläge jeweils während der relativen Refraktärzeit erfolgt.

Im Gegensatz zu Herzschrittmachern nach dem Stand der Technik, bei denen die Dauer des Basis-Intervalls und die Dauer der Reraktärzeit im Zuge der Programmierung durch den behandelnden Arzt getrennt voneinander eingestellt werden müssen, ist im Falle des erfindungsgemäßen Herzschrittmachers in einer ersten Betriebsart vorgesehen, daß die Dauer der Refraktärzeit in Abhängigkeit von der jeweils programmierten Dauer des Basis-Intervalls derart eingestellt wird, daß die Dauer der Refraktärzeit um ein definiertes Maß geringer als die des Basis-Intervalls ist. Dabei ist vorgesehen, daß die Dauer der absoluten Refraktärzeit zwar programmierbar ist, aber einen festen Wert beibehält. Wenn also die Dauer der Refraktärzeit um ein definiertes Maß von beispielsweise 100 ms kürzer als die Dauer des jeweils programmierten Basis-Intervalls ist und eine Dauer der absoluten Refraktärzeit von 125 ms eingestellt ist, wird für eine Dauer des Basis-Intervalls von 1000 ms eine Dauer der Refraktärzeit von 900 ms eingestellt, wobei dann die Dauer der Refraktärzeit 900 ms, die Dauer der relativen Refraktärzeit 775 ms und die Dauer der absoluten Refraktärzeit wie programmiert 125 ms beträgt. Entsprechend ergibt sich für eine programmierte Dauer des Basis-Intervalls von 900 ms eine Dauer der Refraktärzeit von 800 ms, eine Dauer der relativen Refraktärzeit von 675 ms und für die Dauer der absoluten Refraktärzeit wieder der programmierte Wert von 125 ms. Durch die beschriebene Maßnahme wird bei geeigneter, dem jeweiligen Behandlungsfall angepaßter Wahl des definierten Maßes erreicht, daß einerseits die Refraktärzeit ausreichend lang ist, um zu verhindern, daß Fehldetektionen oder von außen eingestrahlte Störungen ein neues Basis-Intervall in Gang setzen, ohne daß tatsächlich ein spontaner Herzschlag aufgetreten ist, und andererseits die Refraktärzeit nicht so lange dauert, daß das Gerät bei hoher körperlicher Aktivität des Patienten in die asynchrone Betriebsart übergeht, ohne daß dies physiologisch tatsächlich erforderlich ist.

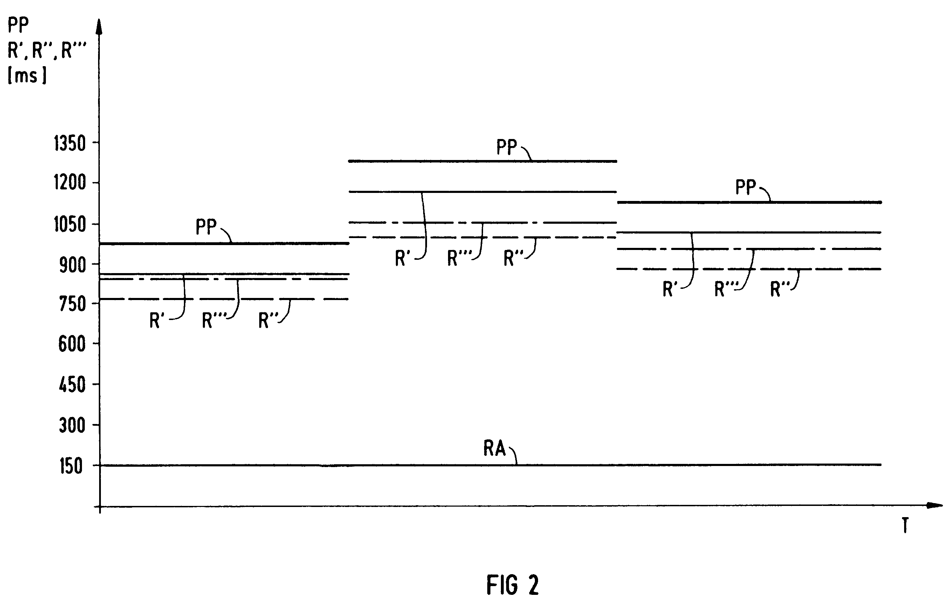

Die der ersten Betriebsart entsprechenden Verhältnisse sind in der Fig. 2 qualitativ für drei unterschiedliche Programmierungen des Herzschrittmachers über der Zeit T dargestellt. Dabei ist zunächst eine Dauer des Basis-Intervalls PP von 975 ms programmiert, die im Zuge einer Umprogrammierung auf 1275 ms verlängert und im Zuge einer weiteren Umprogrammierung auf 1175 ms verkürzt wird. Die Dauer der absoluten Refraktärzeit RA beträgt in allen Fällen 150 ms. Außerdem sind in Fig. 2 für jeweils drei unterschiedliche Werte des definierten Maßes, um das die Refraktärzeit kürzer als die eingestellte Dauer des Basis-Intervalls PP ist, die Refraktärzeiten R′, R'' und R''' eingetragen. Die Refraktärzeiten R′ ergeben sich, wenn die eingestellte Refraktärzeit jeweils um einen definierten Betrag der Zeit, im Falle des in Fig. 2 dargestellten Beispiels 120 ms, kürzer ist als die eingestellte Dauer des Basis-Intervalls PP. Die Refraktärzeiten R'' ergeben sich, wenn die Dauer der Refraktärzeit jeweils um einen definierten Prozentsatz, im Falle des in Fig. 2 dargestellten Beispiels 25%, geringer als die programmierte Dauer des Basis-Intervalls PP ist. Die Refraktärzeiten R''' ergeben sich schließlich, indem die der jeweils programmierten Dauer des Basis-Intervalls PP entsprechende Herzschlagfrequenz um einen definierten Betrag der Frequenz, im Falle des in Fig. 2 dargestellten Beispiels 15 bpm (beats per minute) vergrößert und die entsprechende Periodendauer ermittelt wird, die dann der jeweiligen Refraktärzeit R''' entspricht.

Wie aus der Fig. 1 ersichtlich ist, besitzt der erfindungsgemäße Herzschrittmacher ein mit der Steuerlogik 9 verbundenes Telemetrieregister 19 und einen mit diesem verbundenen Telemetrieschaltkreis 20. Der Herzschrittmacher ist dadurch in der Lage, mit einem nicht dargestellten externen Gerät, einem sog. Programmer bidirektional Daten auszutauschen, was durch den Doppelpfeil 21 angedeutet ist. Es besteht somit die Möglichkeit, den Herzschrittmacher auch nach erfolgter Implantation zu programmieren. Unter anderem können mittels des Programmers über den Telemetrieschaltkreis 20 und das Telemetrieregister 19 die Dauer des Basis-Intervalls und die Dauer der absoluten Refraktärzeit eingestellt werden, wobei in der beschriebenen Weise die entsprechenden Daten über die Datenleitung 17 dem Eingang PRE des PP-Zählers 11 und über die Datenleitung 26 dem Eingang PRE des RA-Zählers 13 von der Steuerlogik 9 zugeführt werden. Außerdem besteht die Möglichkeit, das definierte Maß, um das die Dauer der Refraktärzeit geringer als die programmierte Dauer des Basis-Intervalls ist, zu programmieren, und zwar in der Weise, daß das definierte Maß entweder einem definierten Zeitbetrag, einem definierten Prozentsatz oder einem definierten Betrag der Frequenz entspricht, so wie dies im Zusammenhang mit der Fig. 2 beschrieben wurde. Außerdem besteht die Möglichkeit, das definierte Maß jeweils seinem Betrag nach zu programmieren. Die Steuerlogik 9 berechnet dann die dem jeweils programmierten definierten Maß nach Art und Betrag entsprechende Anzahl von Taktimpulsen des Taktgenerators 10 und führt die entsprechenden Daten über eine Datenleitung 24 einer digitalen Rechenschaltung 18 zu. Diese dient dazu, von der der programmierten Dauer des Basis-Intervalls entsprechenden Anzahl von Taktimpulsen des Taktgenerators 10 die dem jeweils programmierten definierten Maß entsprechende Anzahl von Taktimpulsen zu subtrahieren. Als Ausgangsdaten liefert die Rechenschaltung 18 also Daten bezüglich derjenigen Anzahl von Taktimpulsen, die der jeweils einzustellenden Refraktärzeit entspricht. Demnach sind einem ersten Eingang der Rechenschaltung 18 über eine Datenleitung 27 von der Steuerlogik 9 diejenigen Daten zugeführt, die der Dauer des Basis-Intervalls entsprechen. Es handelt sich hierbei in der ersten Betriebsart übrigens um die gleichen Daten, die dem Eingang PRE des PP-Zählers 11 über die Datenleitung 17 zugeführt sind. An einen zweiten Eingang der Rechenschaltung 18 ist die Datenleitung 24 angeschlossen. Der Ausgang der Rechenschaltung 18 ist über eine Datenleitung 25 mit dem Eingang PRE des R-Zählers 12 verbunden. Es wird also deutlich, daß in Abhängigkeit von der Programmierung des Herzschrittmachers die Dauer der Refraktärzeit in der einen oder anderen im Zusammenhang mit Fig. 2 beschriebenen Weise eingestellt wird.

Im Gegensatz zu der vorstehend beschriebenen Betriebsart des Herzschrittmachers, in der die Dauer der Refraktärzeit für eine bestimmte Programmierung des Herzschrittmachers einen festen Wert besitzt, ist im Falle einer zweiten Betriebsart des erfindungsgemäßen Herzschrittmachers, auf die der Herzschrittmacher mittels des Programmers umgeschaltet werden kann, vorgesehen, daß die Dauer der Refraktärzeit in ihrem zeitlichen Verlauf der Periodendauer einer der jeweiligen körperlichen Aktivität des den Herzschrittmacher tragenden Patienten angepaßten Herzschlagfrequenz, deren Ermittlung im folgenden noch beschrieben werden wird, wenigstens im wesentlichen folgt und um ein definiertes Maß geringer als diese Periodendauer ist. Durch diese Maßnahme wird bei geeigneter, dem jeweiligen Behandlungsfall angepaßter Wahl des definierten Maßes erreicht, daß unter Berücksichtigung der momentan vorhandenen körperlichen Aktivität des Patienten einerseits die Refraktärzeit ausreichend lang ist, um zu verhindern, daß Fehldetektionen oder von außen eingestrahlte Störungen ein neues Basis-Intervall in Gang setzen, ohne daß tatsächlich ein spontaner Herzschlag aufgetreten ist, und andererseits die Refraktärzeit nicht so lange dauert, daß das Gerät bei hoher körperlicher Aktivität des Patienten in die asynchrone Betriebsart übergeht, ohne daß dies physiologisch tatsächlich erforderlich ist.

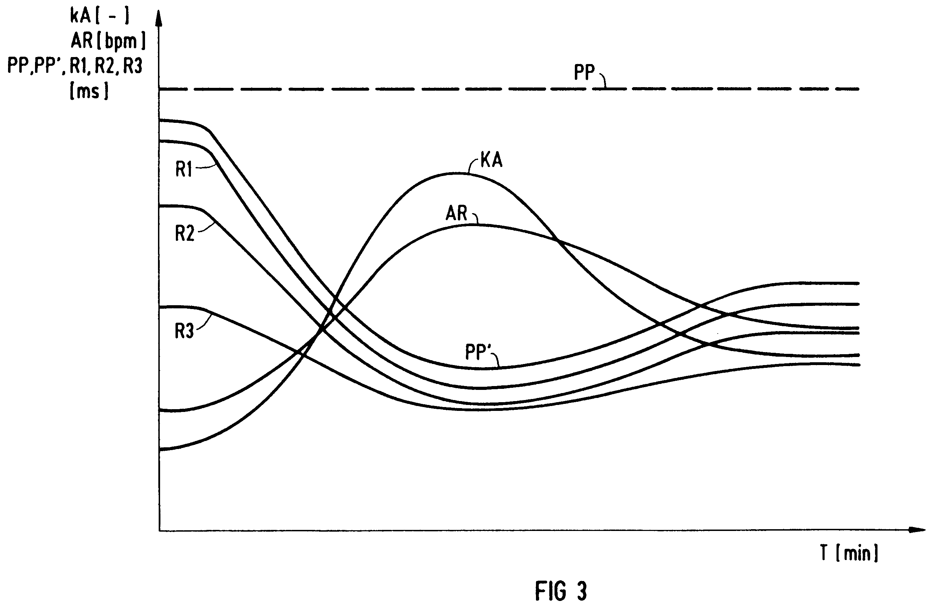

Die der zweiten Betriebsart entsprechenden Verhältnisse sind in der Fig. 3 qualitativ dargestellt. Die Fig. 3 zeigt die Verläufe der körperlichen Aktivität KA eines Patienten ohne Angabe einer Maßeinheit, der dieser körperlichen Aktivität KA angepaßten Herzschlagfrequenz AR in bpm, die der angepaßten Herzschlagfrequenz AR entsprechende Periodendauer PP′ in ms und drei unterschiedliche Verläufe R1, R2 und R3 der Refraktärzeit in ms über der Zeit T in min. Außerdem ist in Fig. 3 die programmierte Dauer des Basis-Intervalls PP in ms strichliert eingetragen, die über der Zeit T konstant ist. Im Falle der Fig. 3 steigt die körperliche Aktivität KA des Patienten von einem relativ niedrigen Niveau aus zunächst an, um dann abzusinken und sich auf einem etwas höheren Niveau zu stabilisieren. Dieser Verlauf der körperlichen Aktivität KA spiegelt sich in den entsprechenden Verläufen der angepaßten Herzschlag frequenz AR und der dieser entsprechenden Periodendauer PP′ wieder. Dies gilt auch für die Verläufe der Refraktärzeit R1, R2 und R3. Der Verlauf R1 der Refraktärzeit ergibt sich, wenn die eingestellte Refraktärzeit jeweils um einen definierten Betrag, im Falle des Beispieles 50 ms, kürzer ist als die der angepaßten Herzschlagfrequenz AR entsprechende Periodendauer PP′. Der Verlauf R2 der Refraktärzeit ergibt sich, wenn die Dauer der Refraktärzeit jeweils um einen definierten Prozentsatz, im Falle des dargestellten Beispieles 20%, geringer als die der angepaßten Herzschlagfrequenz AR entsprechenden Periodendauer PP′ ist. Der Verlauf R3 der Refraktärzeit ergibt sich schließlich, indem die angepaßte Herzschlagfrequenz AR jeweils um einen definierten Betrag der Frequenz, im Falle des Beispieles 50 bpm, vergrößert und die entsprechende Periodendauer ermittelt wird, die der Refraktärzeit R3 entspricht. Die Refraktärzeit R1, R2, R3 ist in allen drei Fällen stets geringer als die programmierte Dauer des Basis-Intervalls PP und die der angepaßten Herzschlagfrequenz AR entsprechende Periodendauer PP′.

Um die der körperlichen Aktivität des Patienten angepaßte Herzschlagfrequenz ermitteln zu können, was die Voraussetzung für die im Zusammenhang mit der Fig. 3 beschriebene Steuerung der Refraktärzeit ist, ist im Falle des erfindungsgemäßen Herzschrittmachers am Ende der in das rechte Ventrikel des Herzens führenden Elektrode 3 ein schematisch angedeuteter Temperatursensor 14 angebracht, der dazu dient, die Temperatur des in dem rechten Ventrikel befindlichen venösen Blutes zu messen, die bekanntlich ein Maß für die körperliche Aktivität des Patienten darstellt. Der Temperatursensor 14 steht über eine parallel zu der Elektrode 3 verlaufende Leitung mit einer Signalaufbereitungsschaltung 15 in Verbindung, von der das mittels des Temperatursensors 14 gewonnene, der körperlichen Aktivität des Patienten entsprechende Signal zu einem Analog/Digital-Wandler 16 gelangt. Ist der Herzschrittmacher auf die Betriebsart gemäß Fig. 3 programmiert, ermittelt die Steuerlogik 9 anhand der ihr über die Datenleitung 29 zugeführten Ausgangsdaten des Analog/Digital-Wandlers 16 die der jeweiligen körperlichen Aktivität des Patienten angepaßte Herzschlagfrequenz nach einem Algorithmus, wie er beispielsweise in der US-PS 4 543 954 beschrieben ist. Die Steuerlogik 9 ermittelt außerdem diejenige Anzahl von Taktimpulsen des Taktgenerators 10, die bei der jeweiligen angepaßten Herzschlagfrequenz zwischen zwei aufeinanderfolgenden Herzschlägen auftritt. Daten bezüglich dieser Anzahl von Taktimpulsen führt die Steuerlogik 9 im Falle der Betriebsart gemäß Fig. 3 anstelle der der Dauer des Basis-Intervalls entsprechenden Daten im Falle der Betriebsart gemäß Fig. 2 über die Datenleitung 27 dem ersten Eingang der Rechenschaltung 18 zu, die im Falle der Betriebsart gemäß Fig. 3 dazu dient, von der genannten Anzahl von Taktimpulsen die Anzahl von Taktimpulsen zu subtrahieren, die dem jeweils gewählten definierten Maß entspricht, um das die Dauer der Refraktärzeit geringer sein soll als die der ermittelten angepaßten Herzschlagfrequenz entsprechende Periodendauer. Die Daten bezüglich derjenigen Anzahl von Taktimpulsen, die dem jeweiligen definierten Maß nach Art und Betrag entspricht, führt die Steuerlogik 9 dem zweiten Eingang der Rechenschaltung 18 wieder über die Datenleitung 24 zu. Die der Differenz der beiden Anzahlen von Taktimpulsen entsprechenden Ausgangsdaten der elektronischen Rechenschaltung 18 sind, wie schon erläutert wurde, dem Eingang PRE des R-Zählers 12 über die Datenleitung 25 zugeführt. Es erfolgt somit eine Steuerung der Dauer der Refraktärzeit in der im Zusammenhang mit Fig. 3 erläuterten Weise in Abhängigkeit von der körperlichen Aktivität des Patienten, wobei die in Fig. 3 nicht eingetragene absolute Refraktärzeit nach erfolgter Programmierung des Gerätes ebenso wie das Basis-Intervall PP eine konstante Dauer besitzt.

In einer dritten Betriebsart des Herzschrittmachers, auf die dieser mittels des Programmers umgeschaltet werden kann und die ansonsten der zweiten Betriebsart entspricht, führt die Steuerlogik 9 dem Eingang PRE des PP-Zählers 11 über die Datenleitung 17 anstelle der einer fest programmierten Dauer des Basis-Intervalls PP entsprechenden Daten diejenigen Daten zu, die die der Periodendauer PP′ der jeweils ermittelten angepaßten Herzschlagfrequenz AR entsprechende Anzahl von Taktimpulsen angeben. Dies hat zur Folge, daß mittels des Stimulationsimpuls-Generators 8 Herzmuskelkontraktionen im Bereich des Ventrikels mit einer Folgefrequenz stimuliert werden, die der ermittelten angepaßten Herzschlagfrequenz AR entspricht, falls die Folgefrequenz der detektierten spontanen Herzmuskelkontraktionen im Bereich des Ventrikels unter die der körperlichen Aktivität KA des Lebewesens angepaßte Herzschlagfrequenz AR absinkt, da die Dauer des ersten Zeitintervalls, also des Basis-Intervalls, in der dritten Betriebsart gleich der Periodendauer PP′der ermittelten angepaßten Herzschlagfrequenz AR ist. Somit ist zusätzlich sichergestellt, daß die Herzschlagfrequenz des Patienten nicht unter die der jeweiligen körperlichen Aktivität KA des Lebewesens angepaßte Herzschlagfrequenz AR absinken kann. Die angepaßte Herzschlagfrequenz AR ist nach unten durch einen unteren Grenzwert, z.B. 60 bpm, und nach oben durch einen oberen Grenzwert, z.B. 140 bpm, auf einen physiologisch sinnvollen Bereich begrenzt. Die Grenzwerte können den individuellen Bedürfnissen jedes Patienten entsprechend programmiert werden.

Der erfindungsgemäße Herzschrittmacher weist außerdem einen Arrhythmie-Detektor 22 auf, dem das Ausgangssignal der Detektoreinrichtung 6 zugeführt ist. Der Arrhythmie-Detektor 22, ein geeigneter Detektor ist in der US-PS 3 861 387 beschrieben, liefert dann ein Ausgangssignal, wenn eine Herzarrhythmie vorliegt, das Herz 4 also nicht dem Sinusrhythmus folgend schlägt. Das Ausgangssignal des Arrhythmie-Detektors 22 ist außer der Steuerlogik dem Enable-Eingang E eines Zählers 23 zugeführt und deaktiviert den Zähler 23, solange eine Herzarrhythmie vorliegt. Dem Toreingang G des Zählers 23 ist das Ausgangssignal der Detektoreinrichtung 6 zugeführt. Der Takteingang Ck des Zählers 23 ist mit dem Taktgenerator 10 verbunden. Der Zähler 23 zählt also, wenn das Herz 4 dem Sinusrhythmus folgend schlägt, diejenige Anzahl von Taktimpulsen ab, die zwischen zwei aufeinanderfolgenden spontanen Herzschlägen auftritt. Die an dem Ausgang Q des Zählers 23 zur Verfügung stehenden Daten entsprechen also der Zeitdauer zwischen zwei aufeinanderfolgenden spontanen Herzschlägen und stellen somit ein Maß für die spontane Herzschlagfrequenz dar. Solange das Herz 4 dem Sinusrhythmus folgend schlägt, stellen die Ausgangsdaten des Zählers 23 unmittelbar ein Maß für die der jeweiligen körperlichen Aktivität des Lebewesens angepaßte Herzschlagfrequenz dar. Diese Daten sind über eine Datenleitung 29 der Steuerlogik 9 zugeführt, die in der zweiten und dritten Betriebsart des Herzschrittmachers, solange das Ausgangssignal des Arrhythmie-Detektors 22 anzeigt, daß das Herz 4 dem Sinusrhythmus folgend schlägt, diese Daten als der der jeweiligen körperlichen Aktivität des Lebewesens angepaßte Herzschlagfrequenz berücksichtigt Die Ausgangsdaten des Analog/Digital-Wandlers 16 werden also in Perioden, in denen das Herz 4 dem Sinusrhythmus folgend schlägt, von der Steuerlogik 9 nicht zur Ermittlung der der jeweiligen körperlichen Aktivität des Lebewesens angepaßten Herzschlagfrequenz berücksichtigt, und zwar weder im Hinblick auf die Einstellung der Dauer des Basis-Intervalls noch die Dauer der Refraktärzeit. Vielmehr führt die Steuerlogik 9 dem Eingang PRE des PP-Zählers 11 über die Datenleitung 17 und dem ersten Eingang der elektronischen Rechenschaltung 18 über die Datenleitung 27 die Ausgangsdaten des Zählers 23 bzw. aus diesen abgeleitete Daten, z.B. einen zeitlichen Mittelwert, zu. Solange das Herz 4 dem Sinusrhythmus folgend schlägt, wird also die der jeweiligen körperlichen Aktivität des Lebewesens angepaßte Herzschlagfrequenz nicht über einen Algorithmus, der zwangsläufig mit Ungenauigkeiten behaftet ist, sondern durch direkte Messung der jeweils vorliegenden Herzschlagfrequenz ermittelt, was den physiologisch korrektesten Weg darstellt. Somit wird deutlich, daß es sich bei der in Fig. 2 eingetragenen, der jeweiligen körperlichen Aktivität angepaßten Herzschlagfrequenz AR sowohl um die anhand der Ausgangsdaten des Analog/Digital-Wandlers 16 nach einem Algorithmus ermittelte Herzschlagfrequenz als auch um die gemessene tatsächliche Herzschlagfrequenz handeln kann, je nachdem, ob eine Herzarrhythmie vorliegt oder das Herz 4 dem Sinusrhythmus folgend schlägt. Tritt eine Arrhythmie auf, bedarf das Herz 4 also der Stimulation, werden für eine bestimmte Anzahl von aufeinanderfolgenden Stimulationsimpulsen, z.B. 30 Stimulationsimpulse, die zuletzt auf Grundlage der tatsächlich vorliegenden Herzschlagfrequenz eingestellten Dauern des Basis-Intervalls und der Refraktärzeit beibehalten. Ist eine fortlaufende Anzahl von Stimulationsimpulsen erforderlich, die die bestimmte Anzahl überschreitet, zieht die Steuerlogik 9 zur Ermittlung der Dauer des Basis-Intervalls und der Refraktärzeit die Ausgangsdaten des Analog/Digital-Wandlers 16 heran.

Falls der erfindungsgemäße Herzschrittmacher in ein Lebewesen implantiert wird, dessen Herz nur während kurzer Perioden oder überhaupt nicht dem Sinusrhythmus folgend schlägt, oder falls dies aus einem anderen Grund erwünscht ist, besteht die Möglichkeit, durch eine entsprechende Programmierung des Herzschrittmachers zu bewirken, daß die Ausgangsdaten des Zählers 23 grundsätzlich außer Betracht bleiben.

Es ist im Rahmen der Erfindung auch möglich, auf den Arrhythmie-Detektor 23 und den Zähler 24 gänzlich zu verzichten. Es besteht außerdem die Möglichkeit, auch auf den Temperatursensor 14, die Signalaufbereitungsschaltung 15 und den Analog/Digital-Wandler 16 zu verzichten. Der Herzschrittmacher arbeitet dann ausschließlich nach der anhand der Fig. 2 beschriebenen ersten Betriebsart.

Obwohl die Erfindung anhand eines im VVI-Mode arbeitenden Herzschrittmachers beschrieben wurde, kann sie sinngemäß auch bei anderen Herzschrittmachern Anwendung finden. Die Erfindung ist insbesondere im Zusammenhang mit im DDD-Mode arbeitenden Herzschrittmachern von Vorteil, da die hier bestehende Gefahr, daß der Herzschrittmacher infolge von "far field R-oversensing" in den AOO-Mode übergeht, wirksam begegnet ist. Unter "far field R-oversensing" versteht man, daß der QRS-Komplex infolge von vom Ventrikel ausgehender R-Potentialausbreitung durch die atrielle Elektrode erfaßt wird.

Der beschriebene spezielle Aufbau des Herzschrittmachers ist nur beispielhaft zu verstehen. Die für die Erfindung wesentlichen Funktionen können auch bei einem abweichenden Aufbau des Herzschrittmachers realisiert werden.

Die Erfindung wurde vorstehend am Beispiel eines Herzschrittmachers beschrieben. Sie kann jedoch auch bei anderen Gewebestimulatoren eingesetzt werden.

### Bezugszeichenliste

- 1: Gehäuse
- 3: Elektrode
- 4: Herz
- 6: Detektoreinrichtung
- 8: Stimulationsimpuls-Generator
- 9: Steuerlogik
- 10: Taktgenerator
- 11: PP-Zähler
- 12: R-Zähler
- 13: AR-Zähler
- 14: Temperatursensor
- 15: Signalaufbereitungsschaltung
- 16: Analog/Digital-Wandler
- 17: Datenleitung
- 18: Rechenschaltung
- 19: Telemetrieregister
- 20: Telemetrieschaltkreis
- 21: Doppelpfeil
- 22: Arrhythmie-Detektor
- 23: Zähler
- 24, 25, 26, 27, 28, 29: Datenleitung
- AR: angepaßte Kontraktions- bzw. Herzschlagfrequenz
- KA: körperliche Aktivität
- PP: Basis-Intervall, erstes Zeitintervall,
- PP′: Basis-Intervall, Periodendauer
- R′, R'', R''', R1, R2, R3: zweites Zeitintervall, Refraktärzeit
- T: Zeit

## Patentansprüche

1. In den Körper eines Lebewesens implantierbares medizinisches Gerät mit Mitteln (6) zum Detektieren spontaner Herzmuskelkontraktionen, Mitteln (8) zum Stimulieren von Herzmuskelkontraktionen und Steuermitteln (9,10,11,12,13,18), die nach Ablauf eines jeweils durch die Detektion oder die Stimulation einer Herzmuskelkontraktion in Gang gesetzten ersten Zeitintervalls (PP), dessen Dauer einstellbar ist die Mittel (8) zum Stimulieren aktivieren, sofern nicht vor Ablauf des ersten Zeitintervalls (PP) eine ein neues erstes Zeitintervall (PP) in Gang setzende spontane Herzmuskelkontraktion detektiert wird, die verhindern, dass vor Ablauf eines jeweils durch die Detektion oder Stimulation einer Herzmuskelkontraktion in Gang gesetzten zweiten Zeitintervalls (R',R'',R''') eine detektierte spontane Herzmuskelkontraktion ein neues erstes Zeitinterval (PP) in Gang setzt, und die nach einer Detektion einer spontanen Herzmuskelkontraktion während des zweiten Zeitintervalls (R',R''R''') ein neues zweites Zeitintervall (R',R'',R''') in Gang setzen, wobei das zweite Zeitintervall zwei aufeinanderfolgende Zeitabschnitte umfasst und wobei die Dauer des zweiten Zeitintervalls (R',R'',R''') immer um ein definiertes Mass geringer als die eingestellte Dauer des ersten Zeitintervalls (PP) ist, **dadurch gekennzeichnet** dass die Steuermittel (9,10,11,12,13,18) derart ausgebildet sind, dass die Steuermittel (9,10,11,12,13,18) automatisch die Dauer des zweiten Zeitintervalls (R',R'',R''') in abhängigkeit von der eingestellten Dauer des ersten Zeitintervalls (PP) derart einstellen, dass der erste Zeitabschnitt des zweiten Zeitintervalls (R',R'',R''') eine feste Dauer besitzt und der zweite Zeitabschnitt um das definierte Mass verringert wird; dass die Steuermittel (9,10,11,12,13,18) derart ausgebildet sind, dass nur eine während des zweiten Zeitabschnittes des zweiten Zeitintervalls (R',R'',R''') erfolgende Detektion einer spontanen Herzmuskelkontraktion ein neues zweites Zeitintervall (R',R'',R''') in Gang setzt.

2. In den Körper eines Lebewesens implantierbares medizinisches Gerät mit Mitteln (6) zum Detektieren spontaner Herzmuskelkontraktionen, Mitteln (8) zum Stimulieren von Herzmuskelkonträktionen und Steuermitteln (9,10,11,12,13,18), die nach Ablauf eines jeweils durch die Detektion oder die Stimulation einer Herzmuskelkontraktion in Gang gesetzten ersten Zeitintervalls (PP,PP') dessen Dauer einstellbar ist, die Mittel (8) zum Stimulieren aktivieren, sofern nicht vor Ablauf des ersten Zeitintervalls (PP,PP') eine ein neues erstes Zeitintervall (PP,PP,) in Gang setzende spontane Herzmuskelkontraktion detektiert wird, die verhindern, dass vor Ablauf eines jeweils durch die Detektion oder Stimulation einer Herzmuskelkontraktion in Gang gesetzten zweiten Zeitintervalls (R1,R2,R3) eine detektierte spontane Herzmuskelkontraktion ein neues erstes Zeitintervalls (PP,PP') in Gang setzt, und die nach einer Detektion einer spontanen Herzmuskelkontraktion während des zweiten Zeitintervalls (R1,R2,R3) ein neues zweites Zeitintervall (R1,R2,R3) in Gang setzen, wobei das zweite Zeitintervall zwei aufeinanderfolgende Zeitabschnitte umfasst und wobei die Dauer des zweiten Zeitintervalls (R1,R2,R3) immer um ein definiertes Mass geringer als die eingestellte Dauer des ersten Zeitintervalls (PP,PP') ist, **dadurch gekennzeichnet** dass Mittel (9,10,14,15,16,22,23) zur Ermittlung einer an die jeweilige körperliche Aktivität (KA) des Lebewesens angepassten Kontraktionsfrequenz (AR) vorgesehen sind, dass die Steuermittel (9,10,11,12,13,18) derart ausgebildet sind, dass sie die Dauer des ersten Zeitintervalls (PP') so einstellen, dass diese der der ermittelten angepassten Kontraktionsfrequenz (KA) entsprechenden Periodendauer entspricht, dass die genannten Steuermittel (9,10,11,12,13,18) automatisch die Dauer des zweiten Zeitintervalls (R1,R2,R3) derart einstellen, dass diese einerseits in ihrem zeitlichen Verlauf dem zeitlichen Verlauf der der ermittelten angepassten Kontraktionsfrequenz (AR) entsprechenden Periodendauer wenigstens im wesentlichen folgt und geringer als diese Periodendauer ist und andererseits stets geringer als die eingestellte Dauer des ersten Zeitintervalls (PP,PP') ist, dass das zweite Zeitintervall (R1,R2,R3) zwei aufeinanderfolgende Zeitabschnitte umfasst, der erste Zeitabschnitt des zweiten Zeitintervalls (R1,R2,R3) eine feste Dauer besitzt und der zweite Zeitabschnitt um das definierte Mass verringert wird; dass die Steuermittel (9,10,11,12,13,18) derart ausgebildet sind, dass nur eine während des zweiten Zeitabschnittes des zweiten Zeitintervalls (R1,R2,R3) erfolgende Detektion einer spontanen Herzmuskelkontraktion ein neues zweites Zeitintervall (R1,R2,R3) in Gang setzt; und dass die Mittel (9,10,14,15,16,22,23) zur Ermittlung einer der körperlichen Aktivität (KA) des Lebewesens angepassten Kontraktionsfrequenz (AR) die der jeweiligen körperlichen Aktivität (KA) des Lebewesens angepasste Herzschlagfrequenz bzw. die spontane Herzschlagfrequenz des Lebewesens ermitteln.

3. Gerät nach Anspruch 2, **dadurch gekennzeichnet**, dass die Mittel (9,14,15,16,22,23) zur Ermittlung der angepassten Kontraktionsfrequenz (AR) eine Sensoreinrichtung (14) zur Bildung eines der körperlichen Aktivität (KA) entsprechenden Signals aufweisen und dass die Steuermittel (9,10,11,12,13,18) derart ausgebildet sind, dass sie anhand des Signals der Sensoreinrichtung (14) die angepasste Kontraktionsfrepuenz (AR) ermitteln.

4. Gerät nach Anspruch 2 oder 3, **dadurch gekennzeichnet**, dass die Mittel (9,10,14,15,16,19,22,23) zur Ermittlung der angepassten Kontraktionsfrequenz (AR) Mittel (9,10,22,23) zum Bestimmen der Folgefrequenz unmittelbar aufeinanderfolgender Herzmuskelkontraktionen aufweisen und dass die Steuermittel (9,10,11,12,13,18) die bestimmte Folgefrequenz als ermittelte angepasste Kontraktionsfrequenz (AR) berücksichtigen.

5. Gerät nach einem der Ansprüche 2 bis 4, **dadurch gekennzeichnet**, dass die Steuermittel (9,10,11,12,13,18) derart ausgebildet sind, dass sie die Dauer des zweiten Zeitintervalls (R1,R2,R3) so einstellen, dass diese um ein definiertes Mass geringer als die der der ermittelten angepassten Kontraktionsfrequenz (AR) entsprechende Periodendauer ist.

6. Gerät nach Anspruch 1 oder 5, **dadurch gekennzeichnet**, dass das definierte Mass einstellbar ist.

7. Gerät nach einem der Ansprüche 1, 5 oder 6, **dadurch gekennzeichnet**, dass das definierte Mass einem definierten Prozentsatz der Dauer des ersten Zeitintervalls (PP) bzw. der der ermittleten angepassten Kontraktionsfrequenz (AR) entsprechenden Periodendauer entspricht.

8. Gerät nach einem der Ansrpüche 1, 5 oder 6, **dadurch gekennzeichnet**, dass das definierte Mass einem definierten Betrag der Frequenz entspricht, um den die der Dauer des zweiten Zeitintervalls (R''',R3) entsprechende Frequentz grösser als die der Dauer des ersten Zeitintervalls (PP) entsprechende Frequenz bzw. die ermittelte angepasste Kontraktionsfrequenz (AR) ist.

## Claims

1. Medical apparatus which can be implanted in the body of a living being, having means (6) for detecting spontaneous heart muscle contractions, means (8) for stimulating heart muscle contractions, and control means (9, 10, 11, 12, 13, 18) which, at the end of a first time interval (PP), which is started in each case by the detection or the stimulation of a heart muscle contraction and the duration of which can be set, activate the means (8) for stimulating, provided that a spontaneous heart muscle contraction, which starts a new first time interval (PP), is not detected before the end of the first time interval (PP), which prevent a detected spontaneous heart muscle contraction starting a new first time interval (PP) before the end of a second time interval (R', R'', R'''), which is started in each case by the detection or stimulation of a heart muscle contraction, and which, after a detection of a spontaneous heart muscle contraction during the second time interval (R', R'', R'''), start a new second time interval (R', R'', R'''), with the second time interval comprising two successive time sections and with the duration of the second time interval (R', R'', R''') always being shorter by a defined measure than the set duration of the first time interval (PP), characterised in that the control means (9, 10, 11, 12, 13, 18) are constructed in such a way that the control means (9, 10, 11, 12, 13, 18) automatically set the duration of the second time interval (R', R'', R''') as a function of the set duration of the first time interval (PP), in such a way that the first time section of the second time interval (R', R'', R''') has a fixed duration and the second time section is reduced by the defined measure; in that the control means (9, 10, 11, 12, 13, 18) are constructed in such a way that only a detection of a spontaneous heart muscle contraction that takes place during the second time section of the second time interval (R', R'', R''') starts a new time interval (R', R'', R''').

2. Medical apparatus which can be implanted in the body of a living being, having means (6) for detecting spontaneous heart muscle contractions, means (8) for stimulating heart muscle contractions, and control means (9, 10, 11, 12, 13, 18) which, at the end of a first time interval (PP, PP'), which is started in each case by the detection or the stimulation of a heart muscle contraction and the duration of which can be set, activate the means (8) for stimulating, provided that a spontaneous heart muscle contraction, which starts a new first time interval (PP, PP'), is not detected before the end of the first time interval (PP, PP'), which prevent a detected spontaneous heart muscle contraction starting a new first time interval (PP, PP') before the end of a second time interval (R1, R2, R3), which is started in each case by the detection or stimulation of a heart muscle contraction, and which, after a detection of a spontaneous heart muscle contraction during the second time interval (R1, R2, R3), start a new second time interval (R1, R2, R3), with the second time interval comprising two successive time sections and with the duration of the second time interval (R1, R2, R3) always being shorter by a defined measure than the set duration of the first time interval (PP, PP'), characterized in that means (9, 10, 14, 15, 16, 22, 23) for establishing a contraction rate (AR) which is matched to the respective physical activity (KA) of the living being are provided, in that the control means (9, 10, 11, 12, 13, 18) are constructed in such a way that they set the duration of the first time interval (PP') in such a way that the said duration corresponds to the period which corresponds to the established, matched contraction rate (KA (sic)), in that the above-mentioned control means (9, 10, 11, 12, 13, 18) automatically set the duration of the second time interval (R1, R2, R3) in such a way that, on the one hand, in terms of its course with respect to time, the said duration follows at least substantially the course with respect to time of the period corresponding to the established, matched contraction rate (AR) and is shorter than this period, and, on the other hand, is always shorter than the set duration of the first time interval (PP, PP'), in that the second time interval (R1, R2, R3) comprises two successive time sections, the first time section of the second time interval (R1, R2, R3) has a fixed duration and the second time section is reduced by the defined measure; in that the control means (9, 10, 11, 12, 13, 18) are constructed in such a way that only a detection of a spontaneous heart muscle contraction that takes place during the second time section of the second time interval (R1, R2, R3) starts a new second time interval (R1, R2, R3); and in that the means (9, 10, 14, 15, 16, 22, 23) for establishing a contraction rate (AR) which matches the physical activity (KA) of the living being establish the heart beat rate which matches the respective physical activity (KA) of the living being, or the spontaneous heart beat rate of the living being.

3. Apparatus according to claim 2, characterised in that the means (9, 14, 15, 16, 22, 23) for establishing the matched contraction rate (AR) have a sensor device (14) for forming a signal which corresponds to the physical activity (KA), and in that the control means (9, 10, 11, 12, 13, 18) are constructed in such a way that they establish the matched contraction rate (AR) with the aid of the signal of the sensor device (14).

4. Apparatus according to claim 2 or 3, characterised in that the means (9, 10, 14, 15, 16, 19, 22, 23) for establishing the matched contraction rate (AR) have means (9, 10, 22, 23) for determining the repetition rate of directly successive heart muscle contractions, and in that the control means (9, 10, 11, 12, 13, 18) take into account the determined repetition rate as an established, matched contraction rate (AR).

5. Apparatus according to one of the claims 2 to 4, characterized in that the control means (9, 10, 11, 12, 13, 18) are constructed in such a way that they set the duration of the second time interval (R1, R2, R3) in such a way that the said duration is shorter by a defined measure than that of the period corresponding to the established, matched contraction rate (AR).

6. Apparatus according to claim 1 or 5, characterised in that the defined measure can be set.

7. Apparatus according to one of the claims 1, 5 or 6, characterized in that the defined measure corresponds to a defined percentage of the duration of the first time interval (PP) or of the period corresponding to the established, matched contraction rate (AR).

8. Apparatus according to one of the claims 1, 5 or 6, characterized in that the defined measure corresponds to a defined amount of the rate, by which amount the rate corresponding to the duration of the second time interval (R''', R3) is longer than the rate which corresponds to the duration of the first time interval (PP), or the established, matched contraction rate (AR).

## Revendications

1. Appareil médical implantable dans le corps d'un être vivant, comprenant des moyens (6) de détection de contractions spontanées du muscle cardiaque, des moyens (8) de stimulation de contractions du muscle cardiaque et des moyens (9,10,11,12,13,18) de commande, qui, après écoulement d'un premier intervalle de temps (PP) de durée variable commençant lors de la détection ou de la stimulation d'une contraction du muscle cardiaque, activent les moyens (8) de stimulation pour autant qu'avant que le premier intervalle de temps (PP) se soit écoulé, il n'ait pas été détecté une contraction spontanée du muscle cardiaque faisant commencer un nouveau premier intervalle de temps (PP), qui empêchent qu'avant que se soit écoulé un deuxième intervalle de temps (R',R'',R''') commençant lors de la détection ou de la stimulation d'une contraction du muscle cardiaque, une contraction spontanée du muscle cardiaque qui a été détectée ne fasse commencer un nouveau premier intervalle de temps (PP), et qui, après une détection d'une contraction spontanée du muscle cardiaque pendant le deuxième intervalle de temps (R',R'',R'''), font commencer un nouveau deuxième intervalle de temps (R',R'',R'''), le deuxième intervalle de temps comprenant deux laps de temps successifs et la durée du deuxième intervalle de temps (R',R'',R''') étant toujours plus petite d'une valeur définie que la durée réglée du premier intervalle de temps (PP), caractérisé en ce que les moyens (9,10,11,12,13,18) de commande sont tels que les moyens (9,10,11,12,13,18) de commande règlent automatiquement la durée du deuxième intervalle de temps (R',R'',R''') en fonction de la durée réglée du premier intervalle de temps (PP) de façon que le premier laps de temps du deuxième intervalle de temps (R',R'',R''') ait une durée fixe et que le second laps de temps soit diminué de la valeur définie; en ce que les moyens (9,10,11,12,13,18) de commande sont tels que seul une détection d'une contraction spontanée du muscle cardiaque ayant lieu pendant un second laps de temps du deuxième intervalle de temps (R',R'',R''') fasse commencer un nouveau deuxième intervalle de temps (R',R'',R''').

2. Appareil médical implantable dans le corps d'un être vivant, comprenant des moyens (6) de détection de contractions spontanées du muscle cardiaque, des moyens (8) de stimulation de contractions du muscle cardiaque et des moyens (9,10,11,12,13,18) de commande, qui, après écoulement d'un premier intervalle de temps (PP) de durée variable commençant lors de la détection ou de la stimulation d'une contraction du muscle cardiaque, activent les moyens (8) de stimulation pour autant qu'avant que le premier intervalle de temps (PP) se soit écoulé, il n'ait pas été détecté une contraction spontanée du muscle cardiaque faisant commencer un nouveau premier intervalle de temps (PP), qui empêchent qu'avant que se soit écoulé un deuxième intervalle de temps (R',R'',R''') commençant lors de la détection ou de la stimulation d'une contraction du muscle cardiaque, une contraction spontanée du muscle cardiaque qui a été détectée ne fasse commencer un nouveau premier intervalle de temps (PP), et qui, après une détection d'une contraction spontanée du muscle cardiaque pendant le deuxième intervalle de temps (R', R'', R'''), font commencer un nouveau deuxième intervalle de temps (R',R'',R'''), le deuxième intervalle de temps comprenant deux laps de temps successifs et la durée du deuxième intervalle de temps (R',R'',R''') étant toujours plus petite d'une valeur définie que la durée réglée du premier intervalle de temps (PP), caractérisé en ce qu'il est prévu des moyens (9,10,14,15,16,22,23) de détermination d'une fréquence (AR) de contraction adaptée à l'activité (KA) corporelle de l'être vivant, en ce que les moyens (9,10,11,12,13,18) de commande sont tels qu'ils règlent la durée du premier intervalle de temps (PP') de façon que celle-ci correspondent à la durée de la période correspondant à la fréquence (KA) de contraction adaptée qui est déterminée, en ce que lesdits moyens (9,10,11,12,13,18) de commande règlent automatiquement la durée du deuxième intervalle de temps (R1,R2,R3) de façon que cette durée d'une part suive au moins sensiblement dans son écoulement dans le temps, l'écoulement dans le temps de la durée de la période correspondante à la fréquence (AR) de contraction adaptée et déterminée et soit plus petite que cette durée de période et d'autre part soit toujours plus petite que la durée réglée du premier intervalle de temps (PP,PP') en ce que le deuxième intervalle de temps (R1,R2,R3) comprend deux laps de temps successifs, le premier laps de temps du deuxième intervalle (R1,R2,R3) a une durée fixe et le second laps de temps est diminué de la valeur définie; en ce que les moyens (9,10,11,12,13,18) de commande sont tels que seul une détection de contraction spontanée du muscle cardiaque s'effectuant pendant le second laps de temps du deuxième intervalle de temps (R1,R2,R3) fasse débuter un nouveau deuxième intervalle de temps (R1,R2,R3); lorsque les moyens (9,10,14,15,16,22,23) de détermination d'une fréquence (AR) de contraction adaptée à l'activité (KA) corporelle de l'être vivant déterminent la fréquence de battement cardiaque adaptée à l'activité (KA) corporelle de l'être vivant ou la fréquence cardiaque spontanée de l'être vivant.

3. Appareil suivant la revendication 2, caractérisé en ce que les moyens (9,14,15,16,22,23) de détermination de la fréquence (AR) de contraction adaptée comportent un dispositif à capteur (14) de formation d'un signal correspondant à l'activité (KA) corporelle et en ce que les moyens (9,10,11,12,13,18) de commande sont tels qu'ils déterminent, au moyen du signal du dispositif (14) à capteur, la fréquence (AR) de contraction adaptée.

4. Appareil suivant la revendication 2 ou 3, caractérisé en ce que les moyens (9,10,14,15,16,19,22,23) de détermination de la fréquence (AR) de contraction adaptée comportent des moyens (9,10,22,23) de détermination de la fréquence de répétition de contraction du muscle cardiaque se succédant directement et en ce que les moyens (9,10,11,12,13,18) de commande considèrent la fréquence de répétition déterminée comme fréquence (AR) de contraction adaptée qui est déterminée.

5. Appareil suivant l'une des revendications 2 à 4, caractérisé en ce que les moyens (9,10,11,12,13,18) de commande sont tels qu'ils règlent la durée du deuxième intervalle de temps (R1,R2,R3) de façon que celle-ci soit plus petite d'une valeur définie que la durée de la période correspondant à la fréquence (AR) de contraction adaptée qui est déterminée.

6. Appareil suivant l'une des revendications 1 ou 5, caractérisé en ce que la valeur définie est réglable.

7. Appareil suivant l'une des revendications 1, 5 ou 6, caractérisé en ce que la valeur définie correspond à un pourcentage de la durée du premier intervalle de temps (PP) ou de la durée de la période correspondant à la fréquence (AR) de contraction adaptée qui a été déterminée.

8. Appareil suivant l'une des revendications 1, 5 ou 6, caractérisé en ce que la valeur définie représente un montant défini de la fréquence, dont la fréquence, correspondant à la durée du deuxième intervalle de temps (R'',R3), est plus grande que la fréquence correspondant à la durée du premier intervalle du temps (PP) et que la fréquence (AR) de contraction adaptée qui est déterminée.
